# EUROPÄISCHE PATENTSCHRIFT

(11) **EP 0 418 607 B1**
(45) Veröffentlichungstag und Bekanntmachung des Hinweises auf die Patenterteilung: **07.12.1994**
(21) Anmeldenummer: 90116615.7
(22) Anmeldetag: 30.08.1990
(51) Int. Cl.: A61F 13/02, G09F 3/02

(54) **Applikationshilfe für flächige Substratabschnitte**
Application aid for planar sheet sections
Aide pour l'application de parties planes d'une feuille

(30) Priorität: 16.09.1989 DE 3931018
(43) Veröffentlichungstag der Anmeldung: 27.03.1991
(73) Patentinhaber: LTS LOHMANN THERAPIE-SYSTEME GmbH & CO.KG, 56513 Neuwied (DE)
(72) Erfinder: Herrmann, Fritz, Dr. Dipl.-Chem., D-5450 Neuwied 12 (DE); Müller, Walter, Dipl.-Chem., D-5450 Neuwied 1 (DE)
(74) Vertreter: Flaccus, Rolf-Dieter, Dr.

(56) Entgegenhaltungen:
- EP-A- 0 144 891
- EP-A- 0 284 963
- DE-A- 3 307 694
- DE-C- 3 315 271
- GB-A- 2 179 910
- US-A- 2 133 609
- US-A- 3 674 614

## Beschreibung

Die Erfindung betrifft eine Applikationshilfe für auf einem flächenförmigen flexiblen Trägermaterialstreifen mehrfach bevorratete, mechanisch ablösbare, auf dem Trägermaterialstreifen reihenförmig in unmittelbaren Kontakt miteinander oder in einem Abstand von einan der ausgestanzte flächenförmige Substratabschnitte in Form von Schnitten oder Sollbruchlinien im Trägermaterial, sowie ihre Verwendung für flächenförmige therapeutische Systeme, Pflaster oder Etiketten.

Oberflächen oder Oberflächenteile von Substraten müssen oft für die Zeitspanne zwischen Herstellung und Anwendung mit einem Schutz versehen sein. Gründe dafür können beispielsweise in der Aufrechterhaltung der Eigenklebrigkeit, der Empfindlichkeit gegenüber mechanischer Beschädigung oder der Verhinderung des Entweichens flüchtiger Komponenten des Substrates liegen.

Üblicherweise wird ein solcher Schutz der betreffenden Substratflächen durch Kontakt mit einem flächigen Abdeckmaterial erzielt, das im folgenden als Trägermaterial für das Substrat bezeichnet wird.

Das Trägermaterial ist dabei mit dem Substrat durch Adhäsionskräfte verbunden, die durch Einwirkung mechanischer Abzugskräfte überwunden werden können.

Dieser Ablösevorgang, der Transfer des Substratabschnittes zum Applikationsort sowie die Applikation selbst sind problematisch und noch nicht in allen Fällen befriedigend gelöst.

Diese Schritte bereiten besonders dann Schwierigkeiten, wenn die Kontaktfläche des Substratabschnittes mit dem Trägermaterial zwischen Ablösen und Applikation frei von jeglicher Kontamination bleiben muß. Hierbei ist insbesondere erforderlich, eine mechanische Verletzung der Substratkontaktfläche zu vermeiden und/oder ihre Sterilität zu wahren. In manchen Anwendungsfällen kann die Substratkontaktfläche auch reaktive Stoffe enthalten, die erst am Applikationsort ihre Wirkung entfalten sollen. Dies trifft insbesondere bei pharmazeutischen Wirkstoffen zu, die in Pflasterform appliziert eine rein topische Wirkung besitzen.

Eine Problemlösung für haftklebende Folienverbände wird in der EP-A-0 144 891 beschrieben. Danach erlauben zwei Anfaßränder einer Stützfolie das kontaminationsfreie Abziehen der Schutzabdeckung der Haftkleberschicht, den kontaminationsfreien Transfer zum Applikationsort und die kontaminationsfreie Applikation, wobei anschließend die Stützfolie entfernt wird.

Diese Lösung ist jedoch sehr aufwendig. Auf Substratabschnitte in der Größe von Geldmünzen oder auf mehrfachbevorratete Substratabschnitte auf einem Trägermaterialabschnitt ist diese technische Lehre nicht anwendbar. Es ist anzumerken, daß gerade die Mehrfachanordnung von Substratabschnitten auf einem Trägermaterialabschnitt mit abnehmender Größe der Substratabschnitte an Bedeutung gewinnt.

In der europäischen Patentanmeldung EP-A 0 284 963 wird für auf einem flächenförmigen flexiblen Trägermaterialabschnitt mehrfach bevorratete, mechanisch ablösbare flächenformige Substratabschnitte eine Applikationshilfe in Form einer Abziehhilfe vorgeschlagen. Dazu sind in der Kontaktfläche zwischen Substrat und Trägermaterial für jeden Substratabschnitt je ein nicht geradliniger Schnitt bzw. Sollbruchlinie vorgesehen. Durch Anwendung von Druck senkrecht auf das Trägermaterial im Bereich der Kontaktfläche ist ein durch den Schnitt oder die Sollbruchlinie vorgegebener Teil des Trägermaterials in Richtung des Substratabschnittes biegbar und löst dabei den dem Schnitt oder Sollbruchlinie benachbarten Randbereich des Substratabschnittes vom Trägermaterial ab. Damit entsteht eine Anfaßfläche am Substratabschnitt, mit deren Hilfe die völlige Ablösung des Substratabschnittes vom Trägermaterial ermöglicht wird. Hiermit ist zwar das Problem des Ablösens auch von kleineren Substratabschnitten vom Trägermaterial gelöst, doch muß immer noch zumindest ein Teil der Kontaktfläche des Substratabschnittes mit den Fingern oder einem Hilfsinstrument berührt werden, was in vielen Fällen unerwünscht ist.

Aus der US-A-2,133,609 sie liegt dem Obergriff des Anspruchs 1 zugrunde ist eine Anordnung einer Vielzahl von Heftpflastern bekannt, die miteinander zu einem fortlaufenden und aufrollbaren Streifen aneinander befestigt sind. Der Streifen besteht demnach aus einer Serie von einzelnen Pflastern, wobei jedes Bereiche mit Haftmittel aufweist, die ihrerseits mit einzelnen ablösbaren Gazestreifen abgedeckt sind. Bei der Entnahme einzelner Pflaster wird zunächst ein Gazestreifen am freien Ende erfasst und abgezogen. Damit ist die unterhalb des Streifens befindliche Klebschicht freigelegt und muß zwangsläufig beim weiteren Abziehen des Streifens vom nächstfolgenden Streifen angefasst und mit den Fingern berührt werden. Somit ist es unmöglich, den Substratabschnitt kontaminationsfrei vom übrigen Trägermaterial abzuziehen und mit dem Akzeptor in Kontakt zu bringen. Ein die einzelnen Pflaster aufnehmender flächenförmiger flexibler Trägermaterialstreifen ist nicht vorhanden, und eine Applikationshilfe in Form von Schnitten oder Sollbruchlinien fehlt. Die einzelnen Laminate, bestehend aus Trägerabschnitt und Substrat, sind dachschindelförmig übereinander angeordnet und miteinander teilweise verklebt. Dabei ist auch die Herstellung einer derartigen Anordnung umständlich und kostspielig, denn dabei müssen die einzelnen Wundverbandstreifen mit den Wundauflagen zunächst separat hergestellt und dann durch Zusammenkleben mittels Gazestreifen miteinander zu einem zusammenhängenden Gebilde konfektioniert werden.

Die DE-C-33 15 271 beschreibt einen flächenförmigen Laminatabschnitt aus einem Substrat und einer Abdeckung, bei dem der Verbund zwischen Abdeckung und Substrat eine mechanische Wiederablösung der Abdeckung durch Abziehen erlaubt, mit einer Abziehhilfe für die Abdeckung oder Teile der Abdeckung durch angebrachte Schnitte oder Sollbruchlinien, wobei die Schnitte oder Sollbruchlinien in der Abdeckung im Bereich von Biegelinien so vorgesehen sind, daß mindestens der auf der einen Seite der Biegelinie vorgesehene abziehbare Teil der Abdeckung mindestens einen mit ihm zusammenhängenden Teilbereich aufweist, der sich jeweils auf der anderen Seite der Biegelinie befindet und so gestaltet ist, daß er durch das Biegen des Laminatabschnittes längs der Biegelinie aufgrund der relativen Steifigkeit der Abdeckung vom Substrat abgelöst wird und als Abziehhilfe in Form einer Anfaßfläche dient. Wenn dann ein erster Teil der Abdeckfläche abgezogen und damit das Substrat freigelegt ist, muß dieses zur Übertragung auf einen Akzeptor mit den Fingern angefasst werden. Dabei ist ebenfalls eine kontaminationsfreie Übertragung nicht möglich. Die GB-A-21 79 910 zeigt ein Etikett mit einem Deckblatt, das an der Rückseite eine adhäsive Beschichtung aufweist sowie einen das Deckblatt überragenden Träger, wobei in der Kontaktfläche zwischen Deckblatt und Träger ein haarnadelförmiger Schnitt verläuft, der das Etikett in einen größeren Bereich und einen kleineren Bereich unterteilt. Der kleinere Teil des Etiketts ist entlang der Schnitt- bzw. Perforationslinie zum größeren Teil abtrennbar, jedoch wird dabei keine kontaminationsfreie Anfaßfläche freigelegt.

Die Aufgabe der vorliegenden Erfindung ist es daher, eine Applikationshilfe für auf einem flächenförmigen flexiblen Trägermaterialstreifen mehrfach bevorratete, mechanisch ablösbare flächenförmige Substratabschnitte in Form von Schnitten oder Sollbruchlinien im Trägermaterial zu schaffen, die eine kontaminationsfreie Applikation der Substratabschnitte ermöglicht.

Die Lösung dieser Aufgabe wurde nun überraschenderweise darin gefunden, daß für jeden einzelnen Substratabschnitt ein Schnitt oder eine Sollbruchlinie in der Kontaktfläche zwischen Substrat und Trägermaterial verlaufend und die gegenüberliegenden Ränder des Trägermaterialstreifens miteinander verbindend vorgesehen ist und das Trägermaterial die Substratabschnitte wenigstens an einem Ende des Trägermaterialstreifens unter Bildung einer Anfaßhilfe überragt, mit deren Hilfe ein Trägermaterialstreifenteil entlang des benachbarten Schnittes bzw. der benachbarten Sollbruchlinie und der auf diesem haftend bleibende Substratabschnitt kontaminationsfrei vom übrigen Trägermaterial abziehbar und ohne Kontaminationszwang mit dem Akzeptor in Kontakt bringbar ist, und daß die Lage der Schnitte oder Sollbruchlinien so vorgesehen ist, daß die Größe der verbleibenden Kontaktfläche des Substratabschnittes am Anfaßteil des Trägermaterials ausreicht, um die notwendigen Kräfte zum Abziehen des restlichen Teiles des Substratabschnittes aufzunehmen.

Substrat und Trägermaterial sind flächige Gebilde, die jeweils für sich oder auch beide gleichzeitig mehrschichtig ausgebildet sein können. Die Dicke des Laminats kann von Bruchteilen eines Millimeters bis zu mehreren Millimetern betragen. Bevorzugt wird der Zusammenhalt zwischen Substrat und Trägermaterial durch Haftkleber bewerkstelligt, kann aber auch durch andere Kräfte, wie z.B. elektrostatische oder magnetische Kräfte, erzeugt werden.

Gemäß der Erfindung sind auf einem Trägermaterialstreifen ausgestanzte Substratabschnitte reihenförmig in unmittelbarem Kontakt miteinander oder durch form- und/oder produktionsbedingte Gründe in einem gewissen Abstand voneinander aufgebracht. Die Schnitte oder Sollbruchlinien verlaufen ohne Festlegung auf die Geometrie im Trägermaterial selbst im Bereich der Kontaktfläche der Substratabschnitte und des Trägermaterials und verbinden dabei die gegenüberliegenden Ränder des Trägermaterialstreifens. Vorzugsweise stellen die Schnitte oder Sollbruchlinien zwischen sich gegenüberliegenden Rändern des Trägermaterials eine gerade Linie dar. Sie können auch einen nicht linearen Verlauf nehmen.

Dadurch, daß der Trägermaterialstreifen wenigstens an einem Ende die Substratabschnitte überragt, wird eine Anfaßhilfe gebildet, mit deren Hilfe ein Teil des Trägermaterialsstreifens entlang des benachbarten Schnittes bzw. der Sollbruchlinie abgetrennt werden kann. Dabei verbleibt der Substratabschnitt auf diesem Teil haften und kann im übrigen kontaminationsfrei vom Trägermaterial abgezogen werden. Nach kontaminationsfreiem Transfer zum Applikationsort durch Anfassen des überstehenden Teiles des Trägermaterials wird die freiliegende Kontaktfläche des Substratabschnittes ohne einen Kontaminationszwang vor Ort mit dem Akzeptor in Kontakt gebracht, der Rest des Trägermaterials mit Hilfe des überstehenden Teiles problemlos entfernt und der Rest der Substratfläche kontaminationsfrei durch Andrücken in Applikationsstellung gebracht.
Mit der Entnahme des der Anfaßhilfe benachbarten Substratabschnittes wird ein weiterer Teil des Trägermaterialstreifens freigelegt, der nun in gleicher Weise als Anfaßhilfe für den zweiten Substratabschnitt dient.

Diese Vorgehensweise wird bis zur Entfernung des letzten Substratabschnittes auf dem Trägermaterialstreifen fortgesetzt.

Bei der Herstellung braucht daher nur eine einzige Anfaßfläche vorgesehen zu werden, während alle anderen Anfaßflächen für die weitere sukzessive Entfernung der Substratabschnitte sich unabhängig von der Anzahl der Substratabschnitte auf dem Streifen durch Ablösen des jeweils vorhergehenden Substratabschnittes bilden.

Damit wird bei dem Ausstanzen der Substratabschnitte bei einem vollflächigen Laminat von Substrat und Trägermaterial unnötiger Materialabfall vermieden und die notwendige Trägermaterialfläche auf ein Minimum reduziert.

Der Trägermaterialstreifen ist flexibel und kann bei ausreichender Flexibilität des Substrates spulenartig aufgerollt werden und so eine raumsparende Vorratshaltung ermöglichen.

Es bietet sich auch die Möglichkeit, mehrere Trägermaterialstreifen in paralleler Anordnung vorzusehen, die dann an einer zwischen den einzelnen Streifen befindlichen Sollbruchlinie in Einzelstreifen aufgetrennt werden können.

Die im Trägermaterial erforderlichen Schnitte können durch Stanzen, Schneiden, Quetschen oder Prägen oder auch durch Einsatz von Laserstrahlen erzeugt werden.

Für die Bildung der Sollbruchlinien kommen Anstanzen, Perforation, lokale chemische oder thermische Behandlung oder Lasereinwirkung in Frage.

Die Linien können vor oder nach dem Aufbringen des Substrates oder der Substratabschnitte hergestellt werden.

Eine bevorzugte Anwendung des Applikationssystems sind mehrfachbevorratete Substratabschnitte flächenförmiger transdermaler Systeme, Pflaster oder Etiketten.

Die Erfindung wird anhand der nachfolgenden Figuren beispielhaft erläutert. Es zeigen
- Figur 1: die Aufsicht auf einen Abschnitt eines Trägermaterialstreifens mit eckigen, sich berührenden Substratabschnitten,
- Figur 2: den Querschnitt entlang der Linie I/I von Fig.1,
- Figur 3: die Aufsicht auf einen Abschnitt eines Trägermaterialstreifens mit überstehenden Rändern,
- Figur 4: die Aufsicht auf einen Abschnitt eines Trägermaterialstreifens mit runden Substretabschnitten und
- Figur 5: die Aufsicht auf eine Vielfachanordnung von Trägermaterialstreifen mit runden Substratabschnitten.

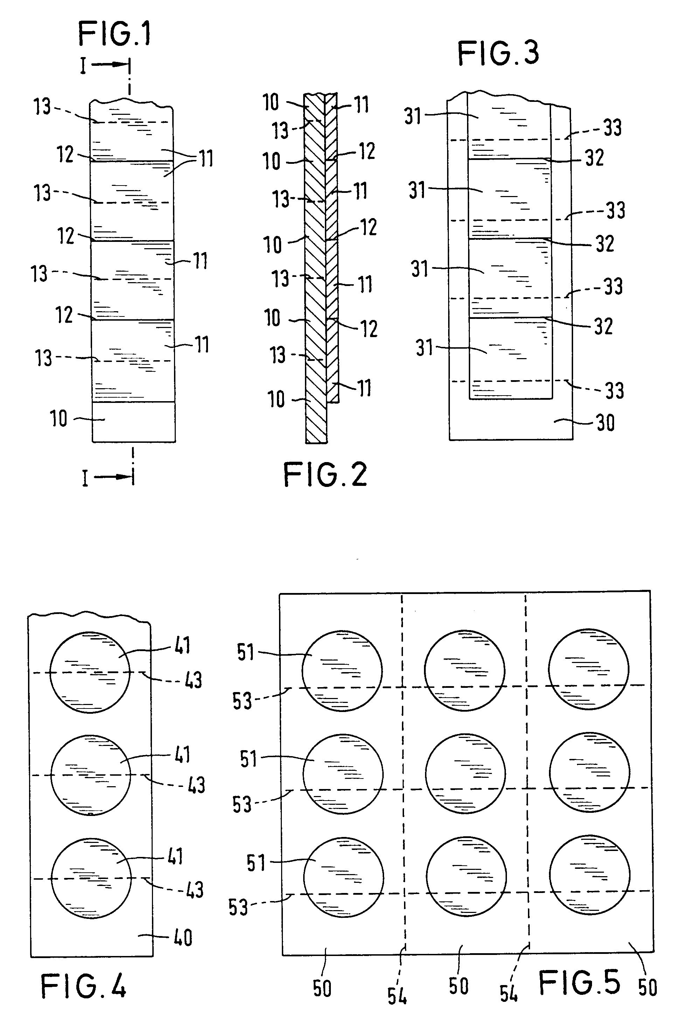

In Figur 1 sind neben den quadratischen sich berührenden Substratabschnitten (11) die Schnitte (12) zwischen den Substratabschnitten zu erkennen. Die Substratabschnitte (11) schließen bündig mit dem Rand des Trägerstreifens (10) ab, der am unteren Ende über die Substratabschnitte herausragt.

Die Schnitte oder Sollbruchlinien im Trägermaterialstreifen sind mit (13) bezeichnet.

Die Figur 2 als Querschnitt durch Figur 1 entlang der Linie I/I verdeutlicht die Gegebenheiten der Figur 1. Es ist deutlich gemacht, daß das Trägermaterial (10) am rechten Ende des Streifens über die Substratabschnitte hinausragt und damit die erste Anfaßfläche bildet. Die mittig unter den Substratabschnitten angebrachten Schnitte oder Sollbruchlinien (13) erlauben das kontaminationsfreie Ablösen der einzelnen Substratabschnitte (11), die durch Schnitte (12) voneinander getrennt sind.

Der Trägermaterialstreifen (30) in Figur 3 überragt die quadratischen Substratabschnitte (31) auch an den Längsrändern des Streifens, wobei auch hier die Substratabschnitte, getrennt durch die Schnitte (32), in engem Kontakt stehen. Die Schnitte oder Sollbruchlinien (33) im Trägermaterial liegen in der unteren Hälfte der Kontaktfläche zwischen Trägermaterial und Substratabschnitt. Die Lage dieser Linien ist so vorzusehen, daß die Größe der verbleibenden Kontaktfläche des Substratabschnittes am Anfaßteil des Trägermaterials ausreicht, um die notwendigen Kräfte zum Abziehen des restlichen Teiles des Substratabschnittes aufzunehmen. Andererseits darf der restliche Teil nicht so klein werden, daß seine Kontaktfläche am Applikationsort nicht ausreicht, um die Kräfte beim Ablösen des noch anhaftenden Trägermaterialabschnittes aufzufangen.

Auch in Figur 4 überragt der Trägermaterialstreifen (40) die runden Substratabschnitte (41), die mit Hilfe der Schnitte oder Sollbruchlinien (43) vom unteren Ende her beginnend nacheinander ohne Kontamination der Kontaktfläche abgelöst und appliziert werden können. Die Substratabschnitte brauchen nicht eckig oder rund zu sein, sondern können alle von der Praxis und dem jeweiligen Applikationszweck geforderten Formen aufweisen. Auch auf dem gleichen Trägermaterialstreifen können sich die geometrischen Formen der Substratabschnitte unterscheiden.

Figur 5 zeigt eine Anordnung, bei der drei Trägermaterialstreifen (50) durch Sollbruchlinien (54) aus einem größeren Trägermaterialabschnitt gebildet werden. Die runden Substratabschnitte (51) sind symetrisch auf den Streifen (50) verteilt und weisen im unteren Drittel ihrer Kontaktfläche in den Trägermaterialstreifen (50) Schnitte oder Sollbruchlinien (53) auf, die die gewünschte Handhabung der Substratabschnitte ermöglichen.

Vor der Applikation der Substratabschnitte werden die Streifen einzeln an den Sollbruchlinien (54) abgetrennt. Die dargestellte Anordnung ist lediglich als Beispiel zur Veranschaulichung des Prinzips der Mehrfachanordnung gewählt.

Aus den Darstellungen in Figur 1 und Figur 3 bis 5 ist ersichtlich, daß die Erfindung eine optimale Nutzung der Fläche des Trägermaterials und Substrates ermöglicht.

## Patentansprüche

1. Applikationshilfe für auf einem flächenförmigen flexiblen Trägermaterialstreifen (10) mehrfach bevorratete, mechanisch ablösbare, auf dem Trägermaterialstreifen (10) reihenförmig in unmittelbarem Kontakt miteinander oder in einem Abstand voneinander ausgestanzte flächenförmige Substratabschnitte (11) in Form von Schnitten oder Sollbruchlinien (13) im Trägermaterial, dadurch gekennzeichnet, daß für jeden einzelnen Substratabschnitt (11) ein Schnitt oder eine Sollbruchlinie (13) in der Kontaktfläche zwischen Substrat (11) und Trägermaterial (10) verlaufend und die gegenüberliegenden Ränder des Trägermaterialstreifens (10) miteinander verbindend vorgesehen ist, daß das Trägermaterial (10) die Substratabschnitte (11) wenigstens an einem Ende des Trägermaterialstreifens (10) unter Bildung einer Anfaßhilfe überragt, mit deren Hilfe ein Trägermaterialstreifenteil entlang des benachbarten Schnittes bzw. der benachbarten Sollbruchlinie (13) und der auf diesem haftend bleibende Substratabschnitt kontaminationsfrei vom übrigen Trägermaterial abziehbar und ohne Kontaminationszwang mit dem Akzeptor in Kontakt bringbar ist, und daß die Lage der Schnitte oder Sollbruchlinien (13) so vorgesehen ist, daß die Größe der verbleibenden Kontaktfläche des Substratabschnittes (11) am Anfassteil der Trägermaterials (10) ausreicht, um die notwendigen Kräfte zum Abziehen des restlichen Teiles des Substratabschnittes (11) aufzunehmen.

2. Applikationshilfe nach Anspruch 1, dadurch gekennzeichnet, daß der Trägermaterial streifen (10) und/oder das Substrat (11) einen mehrschichtigen Aufbau aufweisen.

3. Applikationshilfe nach Anspruch 1 oder 2, dadurch gekennzeichnet, daß der die Substratabschnitte (11) tragende Trägermaterialstreifen (10) spulenartig rollbar ist.

4. Applikationshilfe nach Anspruch 1 oder 2, dadurch gekennzeichnet, daß einzelne Trägermaterialstreifen (10) durch Lösen von Sollbruchlinien (13) aus Vielfachanordnungen erhältlich sind.

5. Applikationshilfe nach einem der Ansprüche 1 bis 4, dadurch gekennzeichnet, daß die Haftung der Substratabschnitte (11) auf dem Trägermaterial streifen (10) durch Haftkleber oder durch elektrostatische oder magnetische Kräfte bewirkt wird.

6. Verwendung der Applikationshilfe nach einem der vorangehenden Ansprüche für flächenförmige therapeutische Systeme, Pflaster oder Etiketten.

## Claims

1. An application aid for mechanically removable, sheet-like substrate sections (11) in the form of cuts or predetermined breaking lines (13) provided in the carrier material, said cuts or predetermined breaking lines being multiply arranged on a sheet-like, flexible strip of carrier material (10), and being punched, in direct contact with or at a distance from each other, in rows, on the strip of carrier material (10), which application aid is characterized in that for each individual substrate section (11) a cut or a predetermined breaking line is provided running in the contact surface between substrate (11) and carrier material (10) and connecting the opposing edges of the strip of carrier material (10),
that the carrier material (10) projects beyond the substrate sections (11) at least at one end of the carrier material strip (10), forming a gripping aid with which a portion of the carrier material strip, and the substrate section which continues to adhere thereto, may be peeled off from the remaining carrier material along the adjacent cut or the adjacent predetermined breaking line (13) in a contamination-free manner and may be brought into contact with the acceptor without automatic contamination,
and that the location of the cuts or predetermined breaking lines (13) is arranged such that the size of the remaining contact area of the substrate section (11) at the gripping tab of the carrier material (10) is sufficient to take up the forces required for pulling off the remaining part of the substrate section (11).

2. The application aid according to claim 1, characterized in that the strip of carrier material (10) and/or the substrate (11) have/has a multi-layered structure.

3. The application aid according to claim 1 or 2, characterized in that the strip of carrier material (10) carrying the substrate sections (11) can be rolled up like a coil.

4. The application aid according to claim 1 or 2, characterized in that individual strips of carrier material (10) can be obtained from multi-arrangements by separation of predetermined breaking lines (13).

5. The application aid according to any one of claims 1 to 4, characterized in that the adhesion of the substrate sections (11) to the carrier material strip (10) is effected by pressure-sensitive adhesives, or by electrostatic or magnetic forces.

6. The use of the application aid according to any one of the preceding claims for sheet-like therapeutic systems, plasters or lables.

## Revendications

1. Aide d'application pour des sections de substrat (11) planiformes, stockées en nombre sur une bande de matériau support (10) flexible plate, pouvant être désolidarisées mécaniquement, réalisées en rangées sur la bande de matériau support (10), en contact direct les unes avec les autres, ou bien estampées individuellement et placées à distance les unes des autres, l'aide d'application se présentant sous forme d'entailles ou de lignes destinées à la rupture (13), ménagées dans le matériau support, caractérisée en ce que, pour chaque section de substrat (11) individuelle, est prévue une entaille ou une ligne destinée à la rupture (13), s'étendant dans la surface de contact entre le substrat (11) et le matériau support (10) et reliant ensemble les bords, placés en regard les uns des autres, de la bande de matériau support (10), en ce que le matériau support (10) dépasse les sections de substrat (11) au moins à une extrémité de la bande de matériau support (10) en constituant une aide à la prise, à l'aide de laquelle une partie de bande de matériau support peut être tirée le long de l'entaille voisine, respectivement de la ligne destinée à la rupture (13) voisine, et la section de substrat subsistant en adhérence sur celle-ci pouvant être extraite du matériau support sans risque de contamination et pouvant être placée en contact avec l'accepteur, sans entraîner obligatoirement de contamination, en ce que la position des entailles ou des lignes destinées à la rupture (13) est prévue telle que la taille de la surface de contact subsistante de la section de substrat (11) sur la partie de saisie du matériau support (10) suffit pour supporter les efforts nécessaires à l'extraction de la partie restante de l'entaille de substrat (11).

2. Aide d'application selon la revendication 1, caractérisée en ce que la bande de matériau support (10) et/ou le substrat (11) présentent une structure multicouche.

3. Aide d'application selon la revendication 1 ou 2, caractérisée en ce que la bande de matériau support (10) portant les sections de substrat (11) peut être enroulée en rouleaux.

4. Aide d'application selon la revendication 1 ou 2, caractérisée en ce que des bandes de matériau support (10) individuelles peuvent être obtenues par désolidarisation des agencements en nombre depuis les lignes destinées à la rupture (13).

5. Aide d'application selon l'une des revendications 1 à 4, caractérisée en ce que l'adhérence des sections de substrat (11) sur la bande de matériau support (10) est provoquée au moyen d'un adhésif, ou bien au moyen d'efforts électrostatiques ou magnétiques.

6. Utilisation de l'aide d'application selon l'une des revendications précédentes pour des systèmes thérapeutiques, des emplâtres ou des étiquettes, planiformes.
